# EUROPEAN PATENT APPLICATION

(11) **EP 4 674 451 A1**
(43) Date of publication of application: **07.01.2026**
(21) Application number: 24207394.8
(22) Date of filing: 18.10.2024
(51) Int. Cl.: A61M 5/20, A61J 1/20

(54) **INJECTION DEVICE**

(30) Priority: 05.07.2024 US 202463667815 P; 17.10.2024 US 202418919381
(71) Applicant: Altek Biotechnology Corporation, 300 Hsinchu City (TW)
(72) Inventor: Yao, Jyun-An, 300 Hsinchu City (TW); Li, Rui-Chang, 300 Hsinchu City (TW); Chu, Yi, 300 Hsinchu City (TW)
(74) Representative: Straus, Alexander

(57) **Abstract**

An injection device (1, 2, 4, 6, 8, 11, 13) includes an injection body (10) and an adaptor (12, 22). An injection end (E1) and a non-injection end (E2) are defined at opposite sides of the injection body (10). The injection body (10) includes an injection member (100), a drug storage member (102), a movable member (104) and a driving mechanism (106). The injection member (100) is located at the injection end (E1) and in fluid communication with the drug storage member (102). The movable member (104) is disposed in the drug storage member (102). The driving mechanism (106) is located at the non-injection end (E2) and engaged with the movable member (104). The adaptor (12, 22) is detachably assembled to the injection end (E1). When the driving mechanism (106) rotates, the movable member (104) linearly moves along a longitudinal axis (LA) of the drug storage member (102).

## Description

### Field of the Invention

The present invention relates to an injection device, particularly an injection device capable of improving injection operation and ensuring injection safety.

### Background of the Invention

Current injectors (e.g. auto-injectors or pen injectors) are used for self-administration of small quantity drugs in a single-dose or multiple-dose manner. Most existing injectors are pure mechanical-based, and are driven by mechanical push against a plunger rod of a cartridge or syringe, respectively. Furthermore, in order to transfer a medical fluid from a vial to the injector, users need to hold the vial with respect to the injector and pull the plunger rod of the injector by manual operation. The manual operation is not much easy for common users and injection safety may be influenced due to misoperation.

### Summary of the Invention

The present invention aims at providing an injection device capable of improving injection operation and ensuring injection safety, thereby resolving the aforesaid problems.

This is achieved by an injection device according to claim 1. The dependent claims pertain to corresponding further developments and improvements.

As will be seen more clearly from the detailed description following below, the claimed injection device includes an injection body and an adaptor. An injection end and a non-injection end are defined at opposite sides of the injection body. The injection body includes an injection member, a drug storage member, a movable member and a driving mechanism. The injection member is located at the injection end and in fluid communication with the drug storage member. The movable member is movably disposed in the drug storage member. The driving mechanism is located at the non-injection end and engaged with the movable member. The adaptor is detachably assembled to the injection end. The injection member protrudes into the adaptor. When the driving mechanism rotates in a first direction, the movable member linearly moves along a longitudinal axis of the drug storage member toward the non-injection end. When the driving mechanism rotates in a second direction, the movable member linearly moves along the longitudinal axis of the drug storage member toward the injection end. The first direction is opposite to the second direction.

In an embodiment, when a drug container is loaded in the adaptor, the injection member penetrates into the drug container. When the driving mechanism engages with and drives the movable member to move linearly along the longitudinal axis of the drug storage member toward the non-injection end, a medical fluid is transferred from the drug container to the drug storage member

In an embodiment, the adaptor includes a sleeve portion for holding the drug container.

In an embodiment, the injection body further includes a shell and a cover. The drug storage member, the movable member and the driving mechanism are disposed in the shell. The injection member extends out of the shell. The cover is movably disposed on the shell relative to the injection member.

In an embodiment, when the adaptor is assembled to the injection end, the adaptor pushes the cover to move toward the non-injection end, such that the injection member is exposed from the cover and protrudes into the adaptor; when the adaptor is disassembled from the injection end, the cover automatically returns to cover the injection member.

In an embodiment, when the injection body is placed on an object by the injection end, the object pushes the cover to move toward the non-injection end, such that the injection member is exposed from the cover and penetrates into the object; when the injection body moves away from the object, the cover automatically returns to cover the injection member and is self-locked.

In an embodiment, the cover includes an extending portion located in the shell. The injection body further includes an injection sensor disposed with respect to the extending portion. When the cover moves toward the non-injection end and the injection sensor senses the extending portion, the driving mechanism drives the movable member to move linearly toward the injection member in response to the injection sensor, such that the injection member injects the medical fluid from the drug storage member to the object.

In an embodiment, the shell includes a first sub-shell and a second sub-shell. The injection member, the drug storage member and the movable member are disposed in the first sub-shell. The driving mechanism is disposed in the second sub-shell. The second sub-shell is detachably engaged with the first sub-shell, and the driving mechanism is detachably engaged with the movable member.

In an embodiment, the shell includes a first sub-shell and a second sub-shell. The driving mechanism includes a driving member and a transmission structure engaged with the driving member. The injection member, the drug storage member, the movable member and the transmission structure are disposed in the first sub-shell. The driving member is disposed in the second sub-shell. The second sub-shell is detachably engaged with the first sub-shell, and the driving member is detachably engaged with the transmission structure.

In an embodiment, the injection body further includes a communication module communicating with an electronic device, and an injection data of the injection device is transmitted to the electronic device through the communication module.

In an embodiment, the injection body further includes an orientation sensor for sensing an orientation of the injection device. When the orientation sensor senses that the injection end is facing up, the driving mechanism drives the movable member to linearly move away from the injection member in response to the orientation sensor.

In an embodiment, the injection body further includes an orientation sensor for sensing an orientation of the injection device. When the orientation sensor senses that the injection end is facing up, the driving mechanism in response to the orientation sensor drives the movable member to linearly move toward the injection member to inject a defined amount of air from the drug storage member into a drug container loaded in the adaptor, and then drives the movable member to linearly move away from the injection member to transfer a medical fluid from the drug container into the drug storage member.

In an embodiment, the injection body further includes an orientation sensor for sensing an orientation of the injection device and a switch for activating the driving mechanism. When the switch is triggered and the orientation sensor senses that the injection end is facing down, the driving mechanism drives the movable member to move linearly toward the injection member in response to the switch and the orientation sensor.

In an embodiment, the adaptor includes an air ventilation and an air needle. The air needle is connected to the air ventilation. When the drug container is loaded in the adaptor, the air needle penetrates into the drug container.

In an embodiment, the movable member includes a first engaging portion, the driving mechanism includes a second engaging portion, and the first engaging portion engages with the second engaging portion, such that the driving mechanism is able to drive the movable member to move linearly toward or away from the injection end.

### Brief Description of the Drawings

In the following, the invention is further illustrated by way of example, taking reference to the accompanying drawings thereof:
FIG. 1 is a schematic view illustrating an injection device according to an embodiment of the invention,
FIG. 2 is a schematic view illustrating a drug container being loaded in an adaptor,
FIG. 3 is a schematic view illustrating a medical fluid 5 being transferred from the drug container to a drug storage member of an injection body,
FIG. 4 is a schematic view illustrating a cover automatically returns to cover an injection member,
FIG. 5 is a schematic view illustrating the injection body being placed on an object by an injection end,
FIG. 6 is a schematic view illustrating the medical fluid being injected into the object,
FIG. 7 is a schematic view illustrating the cover automatically returns to cover the injection member,
FIG. 8 is a schematic view illustrating an injection device according to an embodiment of the invention,
FIG. 9 is a schematic view illustrating an injection device according to an embodiment of the invention,
FIG. 10 is a schematic view illustrating an injection device according to an embodiment of the invention,
FIG. 11 is a schematic view illustrating an injection device according to an embodiment of the invention,
FIG. 12 is a schematic view illustrating the medical fluid being injected into the object,
FIG. 13 is a schematic view illustrating an adaptor according to an embodiment of the invention,
FIG. 14 is a schematic view illustrating an injection device according to an embodiment of the invention,
FIG. 15 is a schematic view illustrating an injection device according to an embodiment of the invention, and
FIG. 16 is a schematic view illustrating an injection device according to an embodiment of the invention.

### Detailed Description

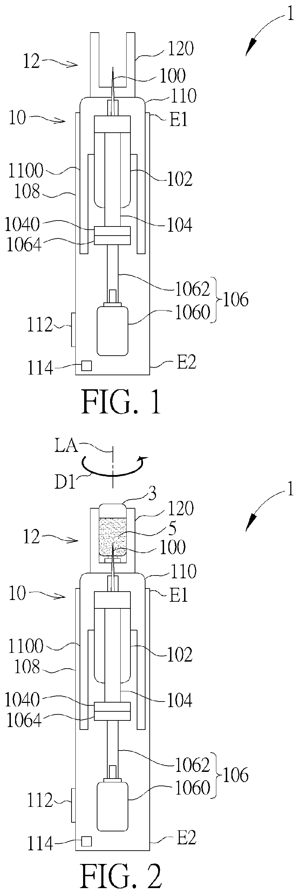

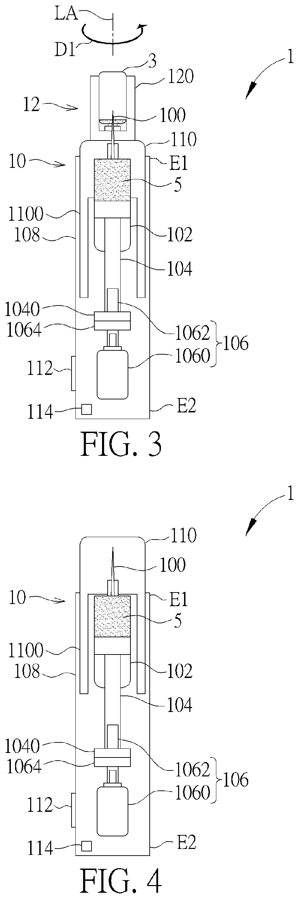

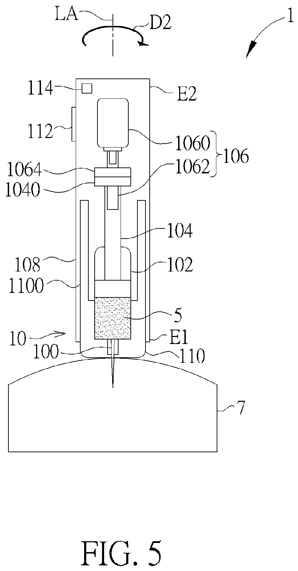

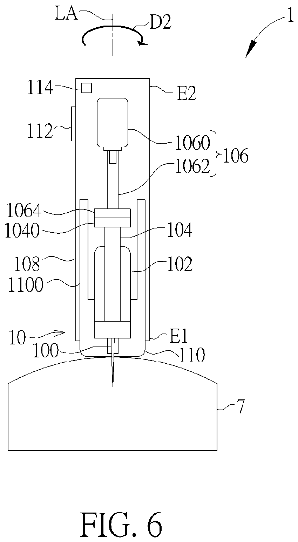

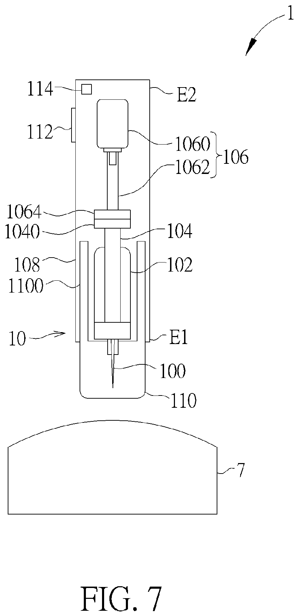

Referring to FIGs. 1 to 7, FIG. 1 is a schematic view illustrating an injection device 1 according to an embodiment of the invention, FIG. 2 is a schematic view illustrating a drug container 3 being loaded in an adaptor 12, FIG. 3 is a schematic view illustrating a medical fluid 5 being transferred from the drug container 3 to a drug storage member 102 of an injection body 10, FIG. 4 is a schematic view illustrating a cover 110 automatically returns to cover an injection member 100, FIG. 5 is a schematic view illustrating the injection body 10 being placed on an object 7 by an injection end E1, FIG. 6 is a schematic view illustrating the medical fluid 5 being injected into the object 7, and FIG. 7 is a schematic view illustrating the cover 110 automatically returns to cover the injection member 100.

As shown in FIG. 1, the injection device 1 comprises an injection body 10 and an adaptor 12, wherein an injection end E1 and a non-injection end E2 are defined at opposite sides of the injection body 10, and the adaptor 12 is detachably assembled to the injection end E1 of the injection body 10. The injection body 10 comprises an injection member 100, a drug storage member 102, a movable member 104 and a driving mechanism 106. The injection member 100 is located at the injection end E1 and in fluid communication with the drug storage member 102. When the adaptor 12 is assembled to the injection end E1 of the injection body 10, the injection member 100 protrudes into the adaptor 12. The movable member 104 is movably disposed in the drug storage member 102. The driving mechanism 106 is located at the non-injection end E2 and engaged with the movable member 104.

In this embodiment, the injection member 100 may be an injection needle or the like, and the drug storage member 102 may be a syringe, a cartridge or the like. The movable member 104 may essentially consist of a plunger and a plunger rod, but the invention is not so limited. The driving mechanism 106 may comprise a driving member 1060 and a transmission structure 1062 engaged with the driving member 1060. The transmission structure 1062 may be engaged with the movable member 104, such that the driving member 1060 may drive the movable member 104 to move linearly through the transmission structure 1062. For example, the driving member 1060 may be a motor and the transmission structure 1062 may essentially consist of a screw track and a screw rod disposed in the screw track. The screw rod may be connected to the motor and the screw track may be engaged with the movable member. Thus, when the motor drives the screw rod to rotate, the screw track will move along the screw rod to drive the movable member 104 to move linearly. In another embodiment, the screw track may also be connected to the motor and the screw rod may also be engaged with the movable member. It should be noted that the transmission structure 1062 is not limited to the aforesaid embodiments. The transmission structure 1062 may be other transmission mechanisms as long as the transmission structure 1062 is able to drive the movable member 104 to move linearly.

In this embodiment, the movable member 104 may comprise a first engaging portion 1040 and the driving mechanism 106 may comprise a second engaging portion 1064. The first engaging portion 1040 engages with the second engaging portion 1064, such that the driving mechanism 106 is able to drive the movable member 104 to move linearly toward or away from the injection end E1 of the injection body 10.

As shown in FIGs. 2 and 3, when the driving mechanism 106 rotates in a first direction D1 (e.g. an output shaft of the driving member 1060 rotates counterclockwise), the movable member 104 may linearly move along a longitudinal axis LA of the drug storage member 102 toward the non-injection end E2. As shown in FIGs. 5 and 6, when the driving mechanism 106 rotates in a second direction D2 (e.g. the output shaft of the driving member 1060 rotates clockwise), the movable member 104 may linearly move along the longitudinal axis LA of the drug storage member 102 toward the injection end E1. The first direction D 1 is opposite to the second direction D2. That is to say, the first direction D 1 may be counterclockwise and the second direction D2 may be clockwise or, alternatively, the first direction D1 may be clockwise and the second direction D2 may be counterclockwise.

As shown in FIG. 1, the injection body 10 may further comprise a shell 108 and a cover 110. In this embodiment, the drug storage member 102, the movable member 104 and the driving mechanism 106 are disposed in the shell 108, and the injection member 100 extends out of the shell 108. The cover 110 is movably disposed on the shell 108 relative to the injection member 100. When the adaptor 12 is assembled to the injection end E1 of the injection body 10, the adaptor 12 pushes the cover 110 to move toward the non-inj ection end E2 of the injection body 10, such that the injection member 100 is exposed from the cover 110 and protrudes into the adaptor 12. At this time, the cover 110 is in a first retraction state. As shown in FIG. 4, when the adaptor 12 is disassembled from the injection end E1 of the injection body 10, the cover 110 may automatically return to cover the injection member 100, so as to prevent a user from being harmed by the injection member 100. At this time, the cover 110 is in a first extension state. In this embodiment, an elastic member (e.g. spring, not shown) may be disposed with respect to the cover 110, such that the elastic member may be compressed by the cover 110 and provide an elastic force for returning the cover 110.

When a user wants to use the injection device 1 to perform an injection process, the user may load a drug container 3 (e.g. vial) in the adaptor 12, as shown in FIG. 2. In this embodiment, the adaptor 12 may comprise a sleeve portion 120 for holding the drug container 3. Furthermore, the sleeve portion 120 may be transparent, allowing users to inspect the drug container 3 loaded in the adaptor 12 and a medical fluid 5 inside the drug container 3.

When the drug container 3 is loaded in the adaptor 12, the injection member 100 penetrates into the drug container 3, as shown in FIG. 2. Then, the user may activate the driving mechanism 106 to drive the movable member 104 to move. In this embodiment, the injection body 10 may further comprise a switch 112 (e.g. button) for activating the driving mechanism 106, wherein the switch 112 may be disposed on a side of the shell 108. In this embodiment, the switch 112 may be coupled to a processing unit 114 through a circuit layout (e.g. circuit board, not shown). In practical applications, the processing unit 114 may be a processor or a controller with data processing function. When the switch 112 is triggered, the processing unit 114 will activate the driving mechanism 106.

As shown in FIG. 3, when the driving mechanism 106 engages with and drives the movable member 104 to move linearly along the longitudinal axis LA of the drug storage member 102 toward the non-injection end E2 of the injection body 10, the medical fluid 5 is transferred from the drug container 3 to the drug storage member 102 through the injection member 100.

Then, the user may disassemble the adaptor 12 with the drug container 3 from the injection body 10. As shown in FIG. 4, when the adaptor 12 is disassembled from the injection end E1 of the injection body 10, the cover 110 may automatically return to cover the injection member 100, so as to prevent the user from being harmed by the injection member 100. Then, the user may place the injection body 10 on an object 7 (e.g. human body) to perform the injection process. As shown in FIG. 5, when the injection body 10 is placed on the object 7 by the injection end E1, the object 7 pushes the cover 110 to move toward the non-injection end E2, such that the injection member 100 is exposed from the cover 110 and penetrates into the object 7. At this time, the cover 110 is in a second retraction state. Then, as shown in FIG. 6, the user may activate the driving mechanism 106 to drive the movable member 104 to move linearly toward the injection member 100, such that the injection member 100 injects the medical fluid 5 from the drug storage member 102 to the object 7.

When the medical fluid 5 is delivered from the drug storage member 102, it means that the injection process has been completed. At this time, the user may remove the injection body 10 from the object 7. As shown in FIG. 7, when the injection body 10 moves away from the object 7, the cover 110 may automatically return to cover the injection member 100, so as to prevent the user from being harmed by the injection member 100. At this time, the cover 110 is in a second extension state. Optionally, when the cover 110 automatically returns to cover the injection member 100, the cover 110 may be self-locked by a self-locking mechanism at the same time. The self-locking mechanism may be achieved by the principle of an automatic ball pen or the like, and it is well known by one skilled in the art, so it will not be depicted in detail herein. In an embodiment, when the cover 110 is self-locked, the cover 110 may not be pushed into the shell 108 again, such that the injection member 100 is hidden in the cover 110 after the injection process is completed. Accordingly, when the user removes the injection body 10 from the object 7, the user will not be harmed by the injection member 100.

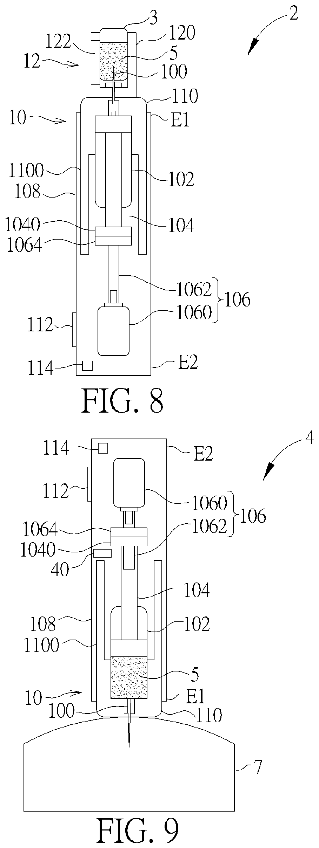

Referring to FIG. 8, FIG. 8 is a schematic view illustrating an injection device 2 according to an embodiment of the invention.

The main difference between the injection device 2 and the aforesaid injection device 1 is that the sleeve portion 120 of the adaptor 12 of the injection device 2 may contain a window 122, as shown in FIG. 8. The window 122 allows users to inspect the drug container 3 loaded in the adaptor 12 and the medical fluid 5 inside the drug container 3.

Referring to FIG. 9, FIG. 9 is a schematic view illustrating an injection device 4 according to an embodiment of the invention.

The main difference between the injection device 4 and the aforesaid injection device 1 is that the injection body 10 of the injection device 4 further comprises an injection sensor 40, as shown in FIG. 9. In this embodiment, the cover 110 comprises an extending portion 1100 located in the shell 108. The injection sensor 40 is also disposed in the shell 108 and disposed with respect to the extending portion 1100. Furthermore, the injection sensor 40 may be coupled to the processing unit 114 through a circuit layout (e.g. circuit board, not shown). The injection sensor 40 is configured to sense the extending portion 1100 to determine whether the injection process is started. As shown in FIG. 9, when the injection body 10 is placed on the object 7 by the injection end E1, the object 7 pushes the cover 110 to move toward the non-injection end E2. When the cover 110 moves toward the non-injection end E2 and the injection sensor 40 senses the extending portion 1100, the driving mechanism 106 may be activated by the processing unit 114 to drive the movable member 104 to move linearly toward the injection member 100 in response to the injection sensor 40, such that the injection member 100 injects the medical fluid 5 from the drug storage member 102 to the object 7. In this embodiment, the injection sensor 40 may be an optical sensor, a magnetic sensor (e.g. Hall sensor), or other object or distance sensors.

According to the aforesaid embodiments, the activation of injection process may be triggered by mechanical, electrical, optical or a combination of the aforesaid mechanisms.

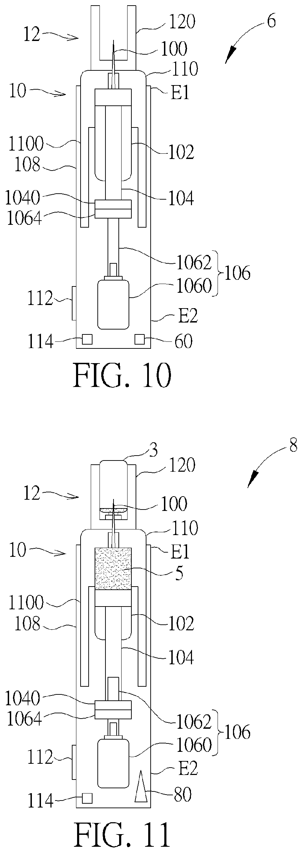

Referring to FIG. 10, FIG. 10 is a schematic view illustrating an injection device 6 according to an embodiment of the invention.

The main difference between the injection device 6 and the aforesaid injection device 1 is that the injection body 10 of the injection device 6 further comprises a communication module 60, as shown in FIG. 10. The communication module 60 may be coupled to the processing unit 114 through a circuit layout (e.g. circuit board, not shown). The communication module 60 is configured to communicate with an electronic device (e.g. server, computer, portable electronic device, etc.), and an injection data (e.g. injection dose, injection time, etc.) of the injection device 6 may be transmitted to the electronic device through the communication module 60, such that a manager, patient's family and/or related person may monitor the injection process through the injection data.

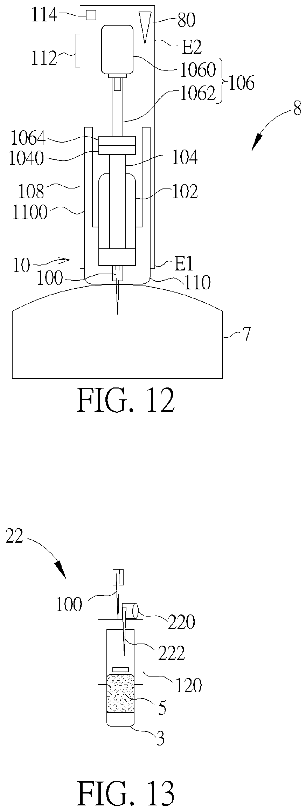

Referring to FIGs. 11 and 12, FIG. 11 is a schematic view illustrating an injection device 8 according to an embodiment of the invention, and FIG. 12 is a schematic view illustrating the medical fluid 5 being injected into the object 7.

The main difference between the injection device 8 and the aforesaid injection device 1 is that the injection body 10 of the injection device 8 further comprises an orientation sensor 80 for sensing an orientation of the injection device 8, as shown in FIG. 11. The orientation sensor 80 may be coupled to the processing unit 114 through a circuit layout (e.g. circuit board, not shown). In this embodiment, when the orientation sensor 80 senses that the injection end E1 of the injection body 10 is facing up (as shown in FIG. 11), it means that the injection body 10 is in a drug transfer mode. At this time, the driving mechanism 106 may be activated by the processing unit 114 to drive the movable member 104 to linearly move away from the injection member 100 in response to the orientation sensor 80, such that the medical fluid 5 is transferred from the drug container 3 to the drug storage member 102 through the injection member 100. Accordingly, the injection device 8 may offer automated operation that replaces manual operation of drug transfer from the drug container 3 to the injection device 8, which allows self-administration by lay persons. In this embodiment, the Orientation sensor 80 allows a defined range of angles for judging the degree of "up" and "down", and the defined range of angles is tunable by software.

Furthermore, when the switch 112 is triggered and the orientation sensor 80 senses that the injection end E1 of the injection body 10 is facing down (as shown in FIG. 12), it means that the injection body 10 is in an injection mode. At this time, the driving mechanism 106 may be activated by the processing unit 114 to drive the movable member 104 to move linearly toward the injection member 100 in response to the switch 112 and the orientation sensor 80, such that the injection member 100 injects the medical fluid 5 from the drug storage member 102 to the object 7. In this embodiment, the orientation sensor 80 may prevent mix up of drug transfer mode with injection mode and prevent false activation of injection. In addition, the cooperation between the switch 112 and the orientation sensor 80 forms a double-confirmed mechanism to avoid misoperation caused by the switch 112 or the orientation sensor 80.

Referring to FIG. 13, FIG. 13 is a schematic view illustrating an adaptor 22 according to an embodiment of the invention.

The main difference between the adaptor 22 and the aforesaid adaptor 12 is that the adaptor 22 comprises an air ventilation 220 and an air needle 222, as shown in FIG. 13. The air needle 222 is connected to the air ventilation 220. When the drug container 3 is loaded in the adaptor 22, the air needle 222 penetrates into the drug container 3. Thus, the injection member 100 may draw the medical fluid 5 from the drug container 3 directly without need to inject a defined amount of air into the drug container 3 in advance.

Referring to FIG. 8 again, since the adaptor 12 has no air ventilation and air needle, when the orientation sensor 80 senses that the injection end E1 of the injection body 10 is facing up, the driving mechanism 106 in response to the orientation sensor 80 needs to drive the movable member 104 to linearly move toward the injection member 100 first to inject a defined amount of air from the drug storage member 102 into the drug container 3 loaded in the adaptor 12, and then drives the movable member 104 to linearly move away from the injection member 100 to transfer the medical fluid 5 from the drug container 3 into the drug storage member 102. The defined amount of air injected into the drug container 3 is used to balance the pressure within the drug container 3, such that the medical fluid 5 may be transferred from the drug container 3 into the drug storage member 102 smoothly in the following operation.

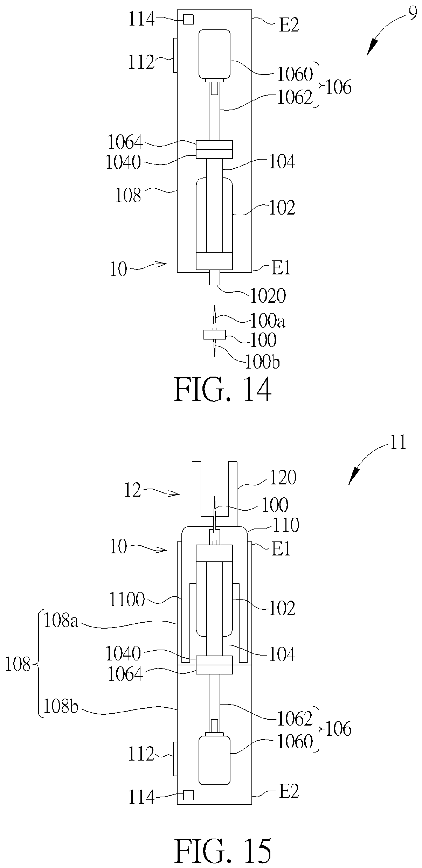

Referring to FIG. 14, FIG. 14 is a schematic view illustrating an injection device 9 according to an embodiment of the invention.

The main difference between the injection device 9 and the aforesaid injection device 1 is that the drug storage member 102 of the injection device 9 comprises a septum 1020, and the injection member 100 of the injection device 9 comprises two needle ends 100a, 100b disposed at opposite sides, as shown in FIG. 14. In this embodiment, one of the two needle ends 100a, 100b penetrates into the septum 1020 to connect the injection member 100 to the drug storage member 102.

Referring to FIG. 15, FIG. 15 is a schematic view illustrating an injection device 11 according to an embodiment of the invention.

The main difference between the injection device 11 and the aforesaid injection device 1 is that the shell 108 of the injection device 11 comprises a first sub-shell 108a and a second sub-shell 108b, as shown in FIG. 15. In this embodiment, the injection member 100, the drug storage member 102 and the movable member 104 are disposed in the first sub-shell 108a, and the driving mechanism 106 is disposed in the second sub-shell 108b. The second sub-shell 108b is detachably engaged with the first sub-shell 108a, and the driving mechanism 106 is detachably engaged with the movable member 104. Thus, the first sub-shell 108a with related components and the second sub-shell 108b with related components may be detached from each other after injection, wherein the first sub-shell 108a with related components may be disposable after injection, whereas the second sub-shell 108b with related components may be disposable or reusable after injection.

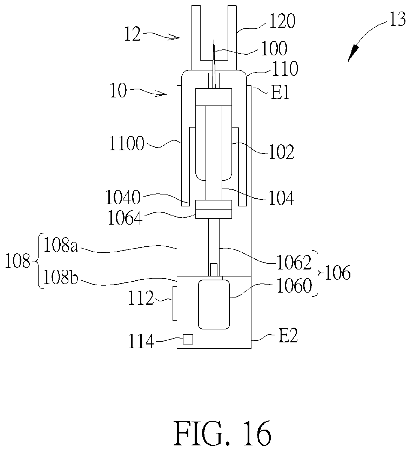

Referring to FIG. 16, FIG. 16 is a schematic view illustrating an injection device 13 according to an embodiment of the invention.

The main difference between the injection device 13 and the aforesaid injection device 1 is that the shell 108 of the injection device 13 comprises a first sub-shell 108a and a second sub-shell 108b, as shown in FIG. 16. In this embodiment, the injection member 100, the drug storage member 102, the movable member 104 and the transmission structure 1062 of the driving mechanism 106 are disposed in the first sub-shell 108a, and the driving member 1060 of the driving mechanism 106 is disposed in the second sub-shell 108b. The second sub-shell 108B is detachably engaged with the first sub-shell 108a, and the driving member 1060 is detachably engaged with the transmission structure 1062. Thus, the first sub-shell 108a with related components and the second sub-shell 108b with related components may be detached from each other after injection, wherein the first sub-shell 108a with related components may be disposable after injection, whereas the second sub-shell 108b with related components may be disposable or reusable after injection.

It should be noted that all of the aforesaid embodiments may be implemented individually or in combination according to practical applications.

As mentioned in the above, the injection device of the invention utilizes the driving mechanism to drive the movable member to move along the longitudinal axis of the drug storage member, so as to achieve automated injection of drug. Furthermore, the injection device of the invention is equipped with the adaptor for loading the drug container, such that the medical fluid can be transferred from the drug container to the drug storage member automatically by activating the driving mechanism. Thus, users neither need to hold the drug container with respect to the injection body, nor need to pull or push the movable member to move by manual operation. Accordingly, the injection device of the invention allows easy and automated drug transfer from the drug container to the injection device and allows automated injection of drug with minimal manual operation, thereby improving injection operation and ensuring injection safety by minimizing human error. Furthermore, the injection device of the invention offers automated operation that replaces manual operation of drug transfer from the drug container to the injection device, which allows self-administration by lay persons.

## Claims

1. An injection device (1, 2, 4, 6, 8, 11, 13) **characterized by** the injection device (1, 2, 4, 6, 8, 11, 13) comprising:
an injection body (10), an injection end (E1) and a non-injection end (E2) being defined at opposite sides of the injection body (10), the injection body (10) comprising an injection member (100), a drug storage member (102), a movable member (104) and a driving mechanism (106), the injection member (100) being located at the injection end (E1) and in fluid communication with the drug storage member (102), the movable member (104) being movably disposed in the drug storage member (102), the driving mechanism (106) being located at the non-injection end (E2) and engaged with the movable member (104); and
an adaptor (12, 22) detachably assembled to the injection end (E1), the injection member (100) protruding into the adaptor (12, 22);
wherein, when the driving mechanism (106) rotates in a first direction (D1), the movable member (104) linearly moves along a longitudinal axis (LA) of the drug storage member (102) toward the non-injection end (E2); wherein, when the driving mechanism (106) rotates in a second direction (D2), the movable member (104) linearly moves along the longitudinal axis (LA) of the drug storage member (102) toward the injection end (E1); the first direction (D1) is opposite to the second direction (D2).

2. The injection device (1, 2, 4, 6, 8, 11, 13) of claim 1 further **characterized in that** when a drug container (3) is loaded in the adaptor (12, 22), the injection member (100) penetrates into the drug container (3); when the driving mechanism (106) engages with and drives the movable member (104) to move linearly along the longitudinal axis (LA) of the drug storage member (102) toward the non-injection end (E2), a medical fluid (5) is transferred from the drug container (3) to the drug storage member (102).

3. The injection device (1, 2, 4, 6, 8, 11, 13) of claim 1 or 2 further **characterized in that** the adaptor (12, 22) comprises a sleeve portion (120) for holding the drug container (3).

4. The injection device (1, 2, 4, 6, 8, 11, 13) of any of the preceding claims further **characterized in that** the injection body (10) further comprises a shell (108) and a cover (110), the drug storage member (102), the movable member (104) and the driving mechanism (106) are disposed in the shell (108), the injection member (100) extends out of the shell (108), the cover (110) is movably disposed on the shell (108) relative to the injection member (100).

5. The injection device (1, 2, 4, 6, 8, 11, 13) of claim 4 further **characterized in that** when the adaptor (12, 22) is assembled to the injection end (E1), the adaptor (12, 22) pushes the cover (110) to move toward the non-injection end (E2), such that the injection member (100) is exposed from the cover (110) and protrudes into the adaptor (12, 22); when the adaptor (12, 22) is disassembled from the injection end (E1), the cover (110) automatically returns to cover the injection member (100).

6. The injection device (1, 2, 4, 6, 8, 11, 13) of claim 4 further **characterized in that** when the injection body (10) is placed on an object (7) by the injection end (E1), the object (7) pushes the cover (110) to move toward the non-injection end (E2), such that the injection member (100) is exposed from the cover (110) and penetrates into the object (7); when the injection body (10) moves away from the object (7), the cover (110) automatically returns to cover the injection member (100) and is self-locked.

7. The injection device (4) of claim 6 further **characterized in that** the cover (110) comprises an extending portion (1100) located in the shell (108), the injection body (10) further comprises an injection sensor (40) disposed with respect to the extending portion (1100); when the cover (110) moves toward the non-injection end (E2) and the injection sensor (40) senses the extending portion (1100), the driving mechanism (106) drives the movable member (104) to move linearly toward the injection member (100) in response to the injection sensor (40), such that the injection member (100) injects the medical fluid (5) from the drug storage member (102) to the object (7).

8. The injection device (11) of claim 4 further **characterized in that** the shell (108) comprises a first sub-shell (108a) and a second sub-shell (108b), the injection member (100), the drug storage member (102) and the movable member (104) are disposed in the first sub-shell (108a), the driving mechanism (106) is disposed in the second sub-shell (108b), the second sub-shell (108b) is detachably engaged with the first sub-shell (108a), and the driving mechanism (106) is detachably engaged with the movable member (104).

9. The injection device (13) of claim 4 further **characterized in that** the shell (108) comprises a first sub-shell (108a) and a second sub-shell (108b), the driving mechanism (106) comprises a driving member (1060) and a transmission structure (1062) engaged with the driving member (1060); the injection member (100), the drug storage member (102), the movable member (104) and the transmission structure (1062) are disposed in the first sub-shell (108a), the driving member (1060) is disposed in the second sub-shell (108b), the second sub-shell (108b) is detachably engaged with the first sub-shell (108a), and the driving member (1060) is detachably engaged with the transmission structure (1062).

10. The injection device (6) of any of the preceding claims further **characterized in that** the injection body (10) further comprises a communication module (60) communicating with an electronic device, and an injection data of the injection device (6) is transmitted to the electronic device through the communication module (60).

11. The injection device (8) of any of the preceding claims further **characterized in that** the injection body (10) further comprises an orientation sensor (80) for sensing an orientation of the injection device (8); when the orientation sensor (80) senses that the injection end (E1) is facing up, the driving mechanism (106) drives the movable member (104) to linearly move away from the injection member (100) in response to the orientation sensor (80).

12. The injection device (8) of any of claims 1 to 10 further **characterized in that** the injection body (10) further comprises an orientation sensor (80) for sensing an orientation of the injection device (8); when the orientation sensor (80) senses that the injection end (E1) is facing up, the driving mechanism (106) in response to the orientation sensor (80) drives the movable member (104) to linearly move toward the injection member (100) to inject a defined amount of air from the drug storage member (102) into a drug container (3) loaded in the adaptor (12), and then drives the movable member (104) to linearly move away from the injection member (100) to transfer a medical fluid (5) from the drug container (3) into the drug storage member (102).

13. The injection device (8) of any of claims 1 to 10 further **characterized in that** the injection body (10) further comprises an orientation sensor (80) for sensing an orientation of the injection device (8) and a switch (112) for activating the driving mechanism (106); when the switch (112) is triggered and the orientation sensor (80) senses that the injection end (E1) is facing down, the driving mechanism (106) drives the movable member (104) to move linearly toward the injection member (100) in response to the switch (112) and the orientation sensor (80).

14. The injection device (1, 2, 4, 6, 8, 11, 13) of any of the preceding claims further **characterized in that** the adaptor (22) comprises an air ventilation (220) and an air needle (222), the air needle (222) is connected to the air ventilation (220); wherein, when the drug container (3) is loaded in the adaptor (12), the air needle (222) penetrates into the drug container (3).

15. The injection device (1, 2, 4, 6, 8, 11, 13) of any of the preceding claims further **characterized in that** the movable member (104) comprises a first engaging portion (1040), the driving mechanism (106) comprises a second engaging portion (1064), and the first engaging portion (1040) engages with the second engaging portion (1064), such that the driving mechanism (106) is able to drive the movable member (104) to move linearly toward or away from the injection end (E1).

## Amended claims

### Amended claims in accordance with Rule 137(2) EPC.

1. An injection device (1, 2, 4, 6, 8, 11, 13) **characterized by** the injection device (1, 2, 4, 6, 8, 11, 13) comprising:
an injection body (10), an injection end (E1) and a non-injection end (E2) being defined at opposite sides of the injection body (10), the injection body (10) comprising an injection member (100), a drug storage member (102), a movable member (104) and a driving mechanism (106), the injection member (100) being located at the injection end (E1) and in fluid communication with the drug storage member (102), the movable member (104) being movably disposed in the drug storage member (102), the driving mechanism (106) being located at the non-injection end (E2) and engaged with the movable member (104); and
an adaptor (12, 22) detachably assembled to the injection end (E1), the injection member (100) protruding into the adaptor (12, 22);
wherein, when the driving mechanism (106) rotates in a first direction (D1), the movable member (104) linearly moves along a longitudinal axis (LA) of the drug storage member (102) toward the non-injection end (E2); wherein, when the driving mechanism (106) rotates in a second direction (D2), the movable member (104) linearly moves along the longitudinal axis (LA) of the drug storage member (102) toward the injection end (E1); the first direction (D1) is opposite to the second direction (D2);
wherein, when a drug container (3) is loaded in the adaptor (12, 22), the injection member (100) penetrates into the drug container (3); when the driving mechanism (106) engages with and drives the movable member (104) to move linearly along the longitudinal axis (LA) of the drug storage member (102) toward the non-injection end (E2), a medical fluid (5) is transferred from the drug container (3) to the drug storage member (102).

2. The injection device (1, 2, 4, 6, 8, 11, 13) of claim 1 further **characterized in that** the adaptor (12, 22) comprises a sleeve portion (120) for holding the drug container (3).

3. The injection device (1, 2, 4, 6, 8, 11, 13) of any of the preceding claims further **characterized in that** the injection body (10) further comprises a shell (108) and a cover (110), the drug storage member (102), the movable member (104) and the driving mechanism (106) are disposed in the shell (108), the injection member (100) extends out of the shell (108), the cover (110) is movably disposed on the shell (108) relative to the injection member (100).

4. The injection device (1, 2, 4, 6, 8, 11, 13) of claim 3 further **characterized in that** when the adaptor (12, 22) is assembled to the injection end (E1), the adaptor (12, 22) pushes the cover (110) to move toward the non-injection end (E2), such that the injection member (100) is exposed from the cover (110) and protrudes into the adaptor (12, 22); when the adaptor (12, 22) is disassembled from the injection end (E1), the cover (110) automatically returns to cover the injection member (100).

5. The injection device (1, 2, 4, 6, 8, 11, 13) of claim 3 further **characterized in that** when the injection body (10) is placed on an object (7) by the injection end (E1), the object (7) pushes the cover (110) to move toward the non-injection end (E2), such that the injection member (100) is exposed from the cover (110) and penetrates into the object (7); when the injection body (10) moves away from the object (7), the cover (110) automatically returns to cover the injection member (100) and is self-locked.

6. The injection device (4) of claim 5 further **characterized in that** the cover (110) comprises an extending portion (1100) located in the shell (108), the injection body (10) further comprises an injection sensor (40) disposed with respect to the extending portion (1100); when the cover (110) moves toward the non-injection end (E2) and the injection sensor (40) senses the extending portion (1100), the driving mechanism (106) drives the movable member (104) to move linearly toward the injection member (100) in response to the injection sensor (40), such that the injection member (100) injects the medical fluid (5) from the drug storage member (102) to the object (7).

7. The injection device (11) of claim 3 further **characterized in that** the shell (108) comprises a first sub-shell (108a) and a second sub-shell (108b), the injection member (100), the drug storage member (102) and the movable member (104) are disposed in the first sub-shell (108a), the driving mechanism (106) is disposed in the second sub-shell (108b), the second sub-shell (108b) is detachably engaged with the first sub-shell (108a), and the driving mechanism (106) is detachably engaged with the movable member (104).

8. The injection device (13) of claim 3 further **characterized in that** the shell (108) comprises a first sub-shell (108a) and a second sub-shell (108b), the driving mechanism (106) comprises a driving member (1060) and a transmission structure (1062) engaged with the driving member (1060); the injection member (100), the drug storage member (102), the movable member (104) and the transmission structure (1062) are disposed in the first sub-shell (108a), the driving member (1060) is disposed in the second sub-shell (108b), the second sub-shell (108b) is detachably engaged with the first sub-shell (108a), and the driving member (1060) is detachably engaged with the transmission structure (1062).

9. The injection device (6) of any of the preceding claims further **characterized in that** the injection body (10) further comprises a communication module (60) communicating with an electronic device, and an injection data of the injection device (6) is transmitted to the electronic device through the communication module (60).

10. The injection device (8) of any of the preceding claims further **characterized in that** the injection body (10) further comprises an orientation sensor (80) for sensing an orientation of the injection device (8); when the orientation sensor (80) senses that the injection end (E1) is facing up, the driving mechanism (106) drives the movable member (104) to linearly move away from the injection member (100) in response to the orientation sensor (80).

11. The injection device (8) of any of claims 1 to 9 further **characterized in that** the injection body (10) further comprises an orientation sensor (80) for sensing an orientation of the injection device (8); when the orientation sensor (80) senses that the injection end (E1) is facing up, the driving mechanism (106) in response to the orientation sensor (80) drives the movable member (104) to linearly move toward the injection member (100) to inject a defined amount of air from the drug storage member (102) into a drug container (3) loaded in the adaptor (12), and then drives the movable member (104) to linearly move away from the injection member (100) to transfer a medical fluid (5) from the drug container (3) into the drug storage member (102).

12. The injection device (8) of any of claims 1 to 9 further **characterized in that** the injection body (10) further comprises an orientation sensor (80) for sensing an orientation of the injection device (8) and a switch (112) for activating the driving mechanism (106); when the switch (112) is triggered and the orientation sensor (80) senses that the injection end (E1) is facing down, the driving mechanism (106) drives the movable member (104) to move linearly toward the injection member (100) in response to the switch (112) and the orientation sensor (80).

13. The injection device (1, 2, 4, 6, 8, 11, 13) of any of the preceding claims further **characterized in that** the adaptor (22) comprises an air ventilation (220) and an air needle (222), the air needle (222) is connected to the air ventilation (220); wherein, when the drug container (3) is loaded in the adaptor (12), the air needle (222) penetrates into the drug container (3).

14. The injection device (1, 2, 4, 6, 8, 11, 13) of any of the preceding claims further **characterized in that** the movable member (104) comprises a first engaging portion (1040), the driving mechanism (106) comprises a second engaging portion (1064), and the first engaging portion (1040) engages with the second engaging portion (1064), such that the driving mechanism (106) is able to drive the movable member (104) to move linearly toward or away from the injection end (E1).
